# EUROPÄISCHE PATENTSCHRIFT

(11) **EP 2 100 763 B1**
(45) Veröffentlichungstag und Bekanntmachung des Hinweises auf die Patenterteilung: **27.10.2010**
(21) Anmeldenummer: 08004620.4
(22) Anmeldetag: 12.03.2008
(51) Int. Cl.: B60K 28/06, B60R 25/04

(54) **Vorrichtung und Verfahren zur Kontrolle einer motorisierten Vorrichtung sowie mobiles Gerät**
Method and device for monitoring a motorised device and mobile device
Procédé et dispositif de contrôle d'un dispositif motorisé and dispositif mobile

(43) Veröffentlichungstag der Anmeldung: 16.09.2009
(73) Patentinhaber: Universität Duisburg-Essen, 45141 Essen (DE)
(72) Erfinder: Jung, Peter, Prof., Dr.-Ing., 47051 Duisburg (DE); Bruck, Guido, Dr., 46562 Voerde (DE)
(74) Vertreter: Zinkler, Franz

(56) Entgegenhaltungen:
- WO-A1-2004/018249
- WO-A1-2005/120878
- DE-B3-102004 005 163
- GB-A- 1 401 318
- GB-A- 2 431 496
- US-B1- 6 229 908
- US-B1- 6 282 475
- US-B1- 7 027 621
- ANONYMOUS: "Führerschein Klasse B Mit 17 - Begleitetes Fahren" INTENET ARTICLE, [Online] Januar 2008 (2008-01), XP002502803 Gefunden im Internet: URL:www.autofahren-mit-17.de> [gefunden am 2008-11-06]
- ANONYMOUS: "Fahren ab 17?" INTERNET ARTICLE, [Online] Juli 2007 (2007-07), XP002502804 Gefunden im Internet: URL:www.begleitetes-fahren.de> [gefunden am 2008-01-06]
- MIKE SIXT: "Nie mehr betrunken die falsche frau anrufen" INTERNET CITATION 30 June 2006 (2006-06-30), pages 1-2, XP002538008 Retrieved from the Internet: URL:http://cgi.cnet.de/alpha/artikel/allge mein/200606/nie-mehr-betrunken- die-falsche-frau-anrufen.htm> [retrieved on 2009-07-21]
- ANONYMOUS: "LG LP4100" INTERNET CITATION 30 June 2006 (2006-06-30), page 1, XP002538013 Retrieved from the Internet: URL:http://www.projectlan.de/galerie/LG_LP 4100-1-7.htm> [retrieved on 2009-07-21]

## Beschreibung

Die vorliegende Erfindung bezieht sich auf den Bereich des Betriebs- und Verkehrsschutzes, insbesondere auf den Bereich der motorisierten Verkehrs- und Arbeits/Betriebsmittel.

Die Betriebssicherheit von Verkehrsmitteln und auch Arbeitsmitteln ist ein bekanntes Problem. Neben vielerlei technischen Aspekten hat sich auch menschliches Fehlverhalten als Ursache von Zwischenfällen bzw. Unfällen erwiesen, das nicht zuletzt als Sicherheitsrisiko eingestuft werden kann. Beispielsweise haben Analysen von Unfällen im Straßenverkehr ergeben, dass es im Wesentlichen drei Unfallursachen gibt, nämlich der Verkehrssituation unangemessen schnelles Fahren, Fahren unter Alkohol- und Drogeneinfluss und Fahren ohne Fahrerlaubnis. Insbesondere die zweite Ursache wirkt sich nicht nur im Bereich des Straßenverkehrs und der Personenbeförderungsmittel aus, sondern ebenso in allen Arbeitsbereichen, in denen beispielsweise mit motorisierten Geräten gearbeitet bzw. umgegangen wird.

So kann es z.B im Bereich von Baustellen, wo Großwerkzeuge, wie Kräne und Bagger usw., eingesetzt werden, ebenfalls aufgrund des Einflusses von Alkohol oder Drogen, nicht zuletzt auch durch Medikamente, zu schwerwiegenden Unfällen kommen.

Bezüglich der Untersuchung auf Alkoholeinfluss lässt sich im Bereich der konventionellen Technik das Alkohol-Interlock-System nennen. Dieses bereits bekannte System ist ein in Fahrzeuge integriertes Konzept, welches alkoholisierte Personen nach einer individuellen Alkoholatemmessung innerhalb der Fahrgastzelle daran hindern soll, den Motor ihres Fahrzeugs zu starten und sich unter Alkoholeinfluss in den Straßenverkehr zu begeben.

Dieses System besteht aus einem Handgerät im Fahrzeuginnenraum, das vergleichbar ist mit Alkomaten, die von der Verkehrsüberwachung oder der Polizei verwendet werden. Hierbei handelt es sich um mobile Geräte, die eine Analyse des Atemalkoholgehalts durchführen können. Beim vorgenannten System ist das Handgerät mit einem Steuergerät zur Datenanalyse bzw. zur Datensicherung unter dem Armaturenbrett verbunden. Das System weist jedoch Schwachstellen auf, z.B. lässt sich das System leicht umgehen, wenn der Fahrzeugführer das System mittels der Atemluft einer zweiten Person manipuliert und so trotz alkoholisiertem Zustand das Fahrzeug starten kann.

Im Bereich der Diebstahlsicherungen bei Fahrzeugen sind mehrere Konzepte bekannt. Eines dieser konventionellen Konzepte stellt die elektronische Wegfahrsperre dar. Hierbei wird ein Starten des Motors verhindert, wenn nicht ein entsprechender Schlüssel oder auch ein elektronischer Chip, wie z.B. durch Nutzung von RFID (RFID = Radio Frequency Identification, Hochfrequenzidentifikation) vorhanden ist. Moderne Konzepte sehen eine Integration eines solchen elektronischen Chips in einen Schlüssel vor, wobei der Chip dann automatisch mit einem Empfänger, der sich im Fahrzeuginneren befindet, kommuniziert. Dabei ist die Diebstahlsicherung fest an das Vorhandensein dieses Schlüssels oder Chips geknüpft. Nachteilig an diesem Konzept ist beispielsweise, dass jeder Besitzer des Schlüssels das Fahrzeug damit leicht betreiben kann, unabhängig davon, ob er der rechtmäßige Eigentümer ist oder nicht.

Ferner ist es nachteilig, dass solche vorbereiteten Schlüssel kostenintensiv sind und meist nur in begrenzter Anzahl vorhanden sind. Wollen mehrere Personen ein Fahrzeug benutzen, so ist es erforderlich, die Schlüssel entsprechend auszutauschen.

Die WO 2005/118326 A1 zeigt ein Konzept zur Verhinderung der Inbetriebnahme eines Kraftfahrzeugs durch einen untauglichen Fahrer. Hier wird, verbunden mit dem Autoschlüssel, ein Alkomat verwendet, der zunächst den Atemalkoholgehalt des Fahrers misst. Nur wenn diese Messung ergibt, dass der Fahrer tauglich ist, sendet der Schlüssel ein Signal, das ein Starten des Fahrzeugs erlaubt. Dabei kann zum einen das Starten des Fahrzeugs verhindert werden, zum anderen kann auch bereits das Öffnen der Türen des Fahrzeugs durch einen untauglichen Fahrer verhindert werden.

Es ist die Aufgabe der vorliegenden Erfindung ein Konzept zur verbesserten Sicherheit einer motorisierten Vorrichtung zu schaffen.

Die Aufgabe wird gelöst durch eine Vorrichtung zur Kontrolle einer motorisierten Vorrichtung gemäß Anspruch 1, ein mobiles Gerät zur Erfassung einer Tauglichkeitsinformation gemäß Anspruch 15 und ein Verfahren zur Kontrolle einer motorisierten Vorrichtung gemäß Anspruch 18.

Die vorliegende Erfindung basiert auf der Erkenntnis, dass die Betriebssicherheit von motorisierten Vorrichtungen, seien es Verkehrsmittel oder auch Betriebsmittel, erheblich erhöht werden kann, wenn der Betreiber verifiziert bzw. identifiziert werden kann. Dies ermöglicht z.B. mit erhöhter Sicherheit festzustellen, ob eine Information, beispielsweise in Form von Atemalkoholwerten, von einem tatsächlichen Fahrer stammt.

Dabei können Ausführungsbeispiele der vorliegenden Erfindung die Möglichkeit bieten, Verifikationen und ferner Identitätsabgleiche durchzuführen, bei denen konventionelle Sensoren zum Einsatz kommen können. Beispielsweise können im Bereich eines Kraftfahrzeugs bereits vorhandene Konzepte mit ausgenutzt werden, so dass zusätzliche Kosten vermieden werden können.

Ausführungsbeispiele der vorliegenden Erfindung können somit den Vorteil bieten, dass zuverlässig gewährleistet werden kann, dass ein Betreiber einer motorisierten Vorrichtung dafür legitimiert ist bzw. dafür auch tauglich ist und nicht unter dem Einfluss von Rauschmitteln steht. Ausführungsbeispiele können ferner den Vorteil aufweisen, dass sie durch Wiederverwendung einer konventionellen Sensorik, wie sie beispielsweise in dem Bereich der Fahrzeugtechnik zum Einsatz kommt, bei Tag und Nacht sowie prinzipiell in einem breiten Temperaturbereich, wie z.B. von -40°C bis +65°C eingesetzt werden können.

Ferner können Ausführungsbeispiele der vorliegenden Erfindung die Möglichkeit bieten, über eine Identifikation eines Betreibers einer motorisierten Vorrichtung, Missbrauch zu unterbinden. Beispielsweise im Bereich der Kfz-Technik und dem öffentlichen Straßenverkehr können Ausführungsbeispiele ferner sicherstellen, dass auch ein Mitbetreiber, wie beispielsweise eine Aufsichtsperson für einen nur unter Aufsicht zugelassenen Fahrer, in einem Kraftfahrzeug präsent ist. Ausführungsbeispiele können ferner ein effizientes Konzept zur Verwaltung von Kraftfahrzeugen bereitstellen, die beispielsweise von mehreren Fahrern benutzt werden können. Die Identifikation eines Fahrers kann neben der Diebstahlsicherung auch die Möglichkeit bieten, auf komplexe Lösungen, wie z.B. Wegfahrsperren, und die damit verbundenen Kosten zu verzichten.

Im Folgenden werden Ausführungsbeispiele der vorliegenden Erfindung anhand der beiliegenden Figuren beschrieben, es zeigen:
- Fig. 1: ein Ausführungsbeispiel einer Vorrichtung zur Kontrolle einer motorisierten Vorrichtung;
- Fig. 2: ein weiteres Ausführungsbeispiel einer Vorrich- tung zur Kontrolle einer motorisierten Vorrich- tung; und
- Fig. 3: einen Innenraum eines Kfz mit einem Ausführungs- beispiel.

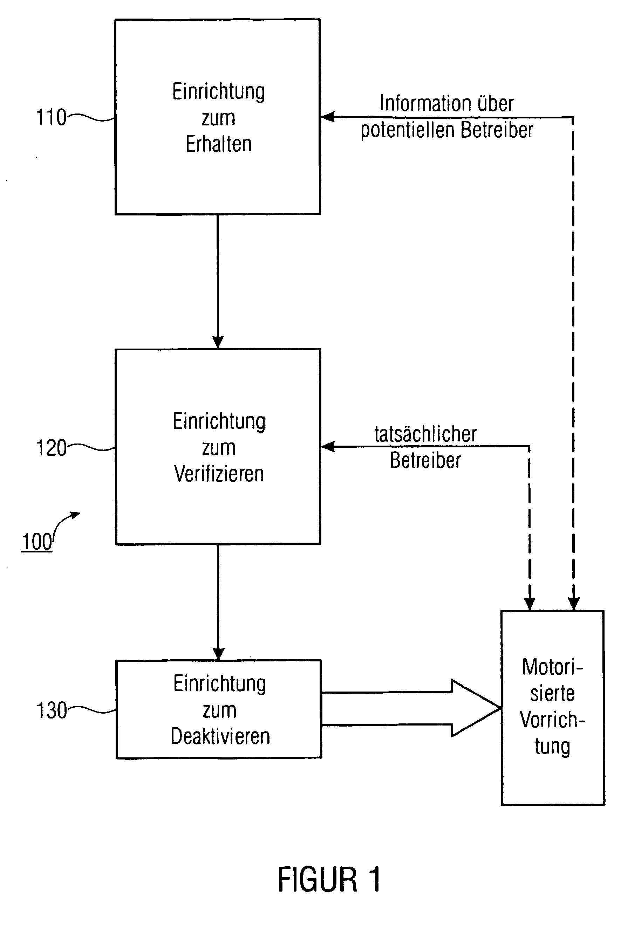

Die Fig. 1 zeigt eine Vorrichtung 100 zur Kontrolle einer motorisierten Vorrichtung mit einer Einrichtung 110 zum Erhalten einer Information über einen potentiellen Betreiber der motorisierten Vorrichtung und einer Einrichtung 120 zum Verifizieren, ob der potentielle Betreiber der motorisierten Vorrichtung ein tatsächlicher Betreiber der motorisierten Vorrichtung sein wird. Die Vorrichtung 100 zur Kontrolle umfasst ferner eine Einrichtung 130 zur Deaktivierung der motorisierten Vorrichtung, wenn eine Diskrepanz zwischen dem potentiellen Betreiber und dem tatsächlichen Betreiber durch die Einrichtung 120 zum Verifizieren festgestellt wird.

Ausführungsbeispiele der Vorrichtung 100 können damit eine Verifikation eines potentiellen Betreibers mit einem tatsächlichen Betreiber durchführen und somit den Betrieb der motorisierten Vorrichtung durch einen nicht berechtigten oder untauglichen potentiellen Betreiber vermeiden. Ausführungsbeispiele können dabei auch von einer Identifikation des potentiellen und/oder des tatsächlichen Betreibers Gebrauch machen.

In einer ersten Gruppe von Ausführungsbeispielen kommt es nicht so sehr auf eine Identifikation des potentiellen Betreibers und des tatsächlichen Betreibers an, vielmehr darauf, dass beide die gleiche Person sind, d.h. auf die Verifikation. In anderen Worten erhält die Einrichtung 110 Informationen über den potentiellen Betreiber, die beispielsweise einen Blutalkoholpegel, der mittels eines Handgeräts ermittelt wurde, umfassen. Die Einrichtung 120 zum Verifizieren ermittelt dann, ob der potentielle Betreiber auch der tatsächliche Betreiber ist. Die Einrichtung 130 zum Deaktivieren deaktiviert die motorisierte Vorrichtung, z. B. das Fahrzeug, wenn potentieller Betreiber und tatsächlicher Betreiber nicht übereinstimmen. Hierdurch kann mittels Ausführungsbeispielen effektiv verhindert werden, dass z. B. ein nüchterner Beifahrer ein Handgerät zur Erfassung eines Blutalkoholpegels bedient, wohingegen der Fahrer einen kritischen Blutalkoholpegelgrenzwert überschreitet. In einem solchen Fall scheitert die Verifikation zwischen potentiellem Betreiber, d. h. Beifahrer, und tatsächlichem Betreiber, d. h. Fahrer.

Die Einrichtung 110 kann in Ausführungsbeispielen ausgebildet sein, um als Information eine Tauglichkeitsinformation des potentiellen Betreibers zu erhalten. Ferner kann die Einrichtung 130 in Ausführungsbeispielen dann ausgebildet sein, um die motorisierte Vorrichtung zu deaktivieren, wenn die Tauglichkeitsinformation eine Untauglichkeit des potentiellen Betreibers anzeigt. Die Einrichtung 110 kann ausgebildet sein, um als Tauglichkeitsinformation eine Information über eine Beeinflussung des Betreibers durch Rauschmittel zu erhalten. Die Information kann dabei eine Information über eine Beeinflussung des potentiellen Betreibers durch Alkohol, Drogen oder auch Medikamente enthalten.

In anderen Worten kann die Einrichtung 110 eine Information darüber erhalten, ob ein potentieller Betreiber unter Einfluss von Rauschmitteln steht, die seine Fähigkeiten, beispielsweise am Straßenverkehr teilzunehmen, d. h. als motorisierte Vorrichtung ein Kraftfahrzeug (Kfz) zu betreiben, beeinträchtigen.

In weiteren Ausführungsbeispielen kann die Einrichtung 120 zum Verifizieren ausgebildet sein, um eine Positionsinformation über den tatsächlichen Betreiber und den potentiellen Betreiber zu erhalten. In anderen Worten kann die Einrichtung 120 zum Verifizieren dazu ausgebildet sein, um festzustellen, ob der potentielle Betreiber, über den die Information vorliegt, sich am gleichen Ort befindet wie der tatsächliche Betreiber. Die Einrichtung 110 kann ausgebildet sein, um mit der Information über den potentiellen Betreiber auch eine Positionsinformation über den potentiellen Betreiber zu erhalten. Prinzipiell kann die Positionsinformation über den potentiellen Betreiber jedoch auch von der Einrichtung 120 zum Verifizieren erhalten und/oder überprüft werden.

Die Einrichtung 120 zum Verifizieren kann in Ausführungsbeispielen dann die Positionsinformation des potentiellen Betreibers mit einer Positionsinformation des tatsächlichen Betreibers vergleichen. Beispielsweise kann die Einrichtung 120 dann, wenn die Positionen des potentiellen Betreibers und des tatsächlichen Betreibers übereinstimmen, verifizieren, dass sich die erhaltenen Informationen auf den tatsächlichen Betreiber beziehen. Die Einrichtung 120 zum Verifizieren kann also ausgebildet sein, um die Positionsinformation des potentiellen Betreibers mit der Positionsinformation des tatsächlichen Betreibers zu vergleichen, und eine Diskrepanz anzuzeigen, wenn die Positionsinformation des potentiellen Betreibers von der Positionsinformation des tatsächlichen Betreibers um mehr als ein vorbestimmtes Maß abweicht. Das vorbestimmte Maß könnte z.B. ½ cm, 1cm, 5cm, 1dm, 5 dm oder auch 1m sein.

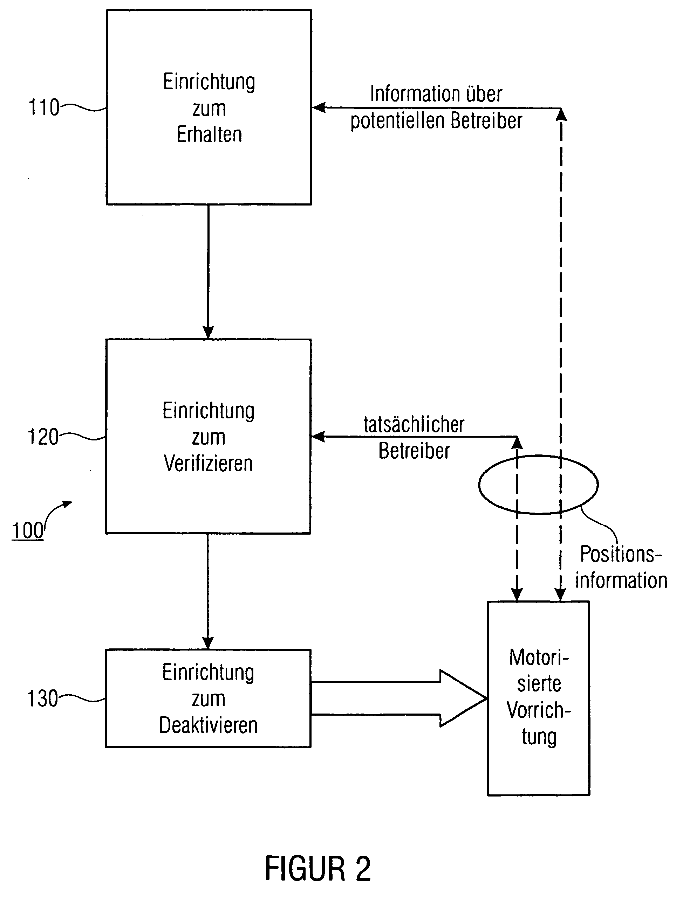

Die Fig. 2 zeigt ein weiteres Ausführungsbeispiel einer Vorrichtung 100 gemäß Fig. 1. In der Fig. 2 ist dargestellt, wie Positionsinformationen über den tatsächlichen Betreiber und den potentiellen Betreiber zwischen der motorisierten Vorrichtung und der Einrichtung 120 bzw. der Einrichtung 110 ausgetauscht werden. Dabei ist es nicht notwendig, dass die Einrichtung 110 zum Erhalten der Information über den potentiellen Betreiber auch dessen Positionsinformation bestimmt, in Ausführungsbeispielen aber denkbar.

In anderen Worten kann die Einrichtung 110 eine Tauglichkeitsinformation eines potentiellen Betreibers erhalten, was beispielsweise durch einen Alkomaten realisiert werden kann. In Ausführungsbeispielen kann nun effektiv verhindert werden, dass eine andere Person den Alkomaten bedient, indem die Einrichtung 120 verifiziert, ob die Position des potentiellen Betreibers, der den Alkomaten bedient, mit der Position des tatsächlichen Betreibers, beispielsweise mit einem Fahrer eines Fahrzeugs, übereinstimmt. Ist dies der Fall und ist die Tauglichkeit bedenklich, so kann die Einrichtung 130 zum Deaktivieren folglich das Kraftfahrzeug deaktivieren.

Die Einrichtung 120 zum Verifizieren kann ausgebildet sein, um die Positionsinformation über den tatsächlichen Betreiber mittels eines ersten Sensors oder auch über einen ersten Sensor zu erhalten. Die Einrichtung 110 kann ausgebildet sein, um die Informationen von einem zweiten Sensor zu erhalten. In Ausführungsbeispielen kann es sich sowohl bei dem ersten als auch bei dem zweiten Sensor um vielfältige Sensoren handeln, die zur Positionsbestimmung oder auch zur Identifikation eines potentiellen bzw. tatsächlichen Betreibers dienen können. Dabei kommen beispielsweise thermische Sensoren, Gewichtssensoren, Drucksensoren, optische Erfassungseinrichtungen, chemische Sensoren oder auch biologische bzw. genetische Sensoren in Betracht. Insbesondere im Bereich der optischen Sensoren ist auch der Einsatz von Fingerabdrucksensoren denkbar.

In Ausführungsbeispielen kann die Einrichtung 120 zum Verifizieren demnach z.B. einen Infrarotsensor oder einen optischen Sensor zur Erzeugung beispielsweise eines Wärmebilds aufweisen. Mit einem Wärmebild lässt sich z.B. überprüfen, ob sich eine Person, die sich auf z. B. dem Fahrersitz befindet, auch diejenige Person ist, die potentieller Betreiber ist, d. h. über die die Einrichtung 110 die Informationen erhält. Beispielsweise wäre es denkbar, ein mobiles Gerät ebenfalls mit dem Wärmebild zu identifizieren und so eine Übereinstimmung im gleichen Bild festzustellen. Die Einrichtung 120 zum Verifizieren kann demnach ausgebildet sein, um eine Verifikation einer Person über eine Wärmebildkamera durchzuführen.

In weiteren Ausführungsbeispielen sind auch herkömmliche Kameras denkbar, wobei auch hier durch entsprechende Bildverarbeitung ein Abgleich zwischen einem potentiellen Betreiber, von dem Informationen z. B. über ein Handgerät abgefragt werden, und einem tatsächlichen Betreiber, der sich z. B. auf einem Fahrersitz befindet, durchgeführt werden. In Ausführungsbeispielen, bei denen es auch auf die Identifikation ankommt, wäre es beispielsweise auch denkbar, dass mittels eines optischen Sensors ein Iris-Scan bei der Person durchgeführt wird, welche sich z. B. auf dem Fahrersitz befindet. Ferner könnte ein Handgerät eine optische oder eine an den jeweiligen Sensor angepasste Kennung aufweisen, die mit Bildverarbeitung oder anderer an den Sensor angepasster Signalverarbeitung leicht detektierbar ist. Schließlich können Ausführungsbeispiele auch Probekörper oder Markierungen einsetzen, anhand derer sich z.B. mobile Handgeräte in einem erfassten Sensorsignal verifizieren bzw. identifizieren lassen.

Die Einrichtung 120 zum Verifizieren wäre somit ebenfalls in der Lage, einen Abgleich der Person auf z. B. einem Fahrersitz mit dem Handgerät durchzuführen und so zu verifizieren, dass derjenige, der das Handgerät bedient, auch derjenige ist, der sich auf dem Fahrersitz eines Fahrzeugs befindet.

Die Einrichtung 110 kann in Ausführungsbeispielen ausgebildet sein, um Informationen schnurlos zu erhalten. Dabei kommen beispielsweise Drahtlosschnittstellen, wie z. B. RFID, Bluetooth, Infrarot oder auch Ultraschall, in Betracht. In Ausführungsbeispielen ist es denkbar, dass ein mobiler Alkomat, oder auch ein anderes mobiles Gerät, das einen anderen Sensor aufweist, der die Tauglichkeit oder Identifikation eines Fahrers erlaubt, mit einer Schnurlosschnittstelle ausgestattet ist. Eine Kommunikation mit diesem mobilen Gerät könnte beispielsweise derart vorgesehen sein, dass sie nur an einem bestimmten Ort, z. B. im Bereich des Cockpits eines Kfz, funktionieren kann. Befindet sich das mobile Gerät in diesem Bereich, so kann eine Messung beginnen und eine entsprechende Kommunikation über eine solche Schnittstelle stattfinden.

In einem solchen Fall könnte beispielsweise ein Gewichtssensor im Fahrersitz sicherstellen, dass eine Person nach der Messung, während der sich das mobile Gerät in dem besagten Bereich befand, den Fahrersitz nicht mehr verlässt. In anderen Worten, für den Fall, dass der Gewichtssensor ein Verlassen des Fahrersitzes festgestellt, scheitert die Verifikation der Einrichtung 120 zum Verifizieren und die Einrichtung 130 zum Deaktivieren deaktiviert das Fahrzeug. Auf diese Art und Weise könnte beispielsweise sichergestellt werden, dass ein Fahrer tauglich ist. Ein Deaktivieren kann dann auch direkt, wenn die Untauglichkeit des potentiellen Betreibers festgestellt wird, erfolgen.

In einem weiteren Ausführungsbeispiel könnte sich an dem mobilen Gerät ein Fingerabdrucksensor befinden, sowie z.B. an einem Fahrersitz. Der Fingerabdrucksensor am Fahrersitz könnte sich beispielsweise zwischen Fahrersitz und Fahrertür befinden, wobei in einem weiteren Ausführungsbeispiel weitere Voraussetzung sein könnte, dass die Fahrertür geschlossen ist. Der Fingerabdrucksensor wäre dann nur von einem Fahrer, d.h. einer sich auf dem Fahrersitz befindlichen Person leicht zu erreichen, nicht jedoch von Personen, die sich an anderen Plätzen innerhalb des Fahrzeuges befinden. Über den Fingerabdrucksensor am Fahrersitz kann der Fahrer identifiziert werden, über den Fingerabdrucksensor am mobilen Gerät kann der Fahrer ebenfalls identifiziert werden. Ein Abgleich der Fingerabdrücke durch die Einrichtung 120 zum Verifizieren kann nun eine Tauglichkeit des Fahrers im Zusammenspiel mit Tauglichkeitsinformation feststellen, bzw. den Fahrer identifizieren und optional mit einer Datenbank abgleichen.

Prinzipiell können Fingerabdrucksensoren aber auch an anderen Orten angebracht sein, z. B. wäre es denkbar, auch im Bereich eines Lenkrads usw. einen Fingerabdrucksensor anzubringen, so dass auch während der Fahrt überprüft werden kann, dass kein Austausch zwischen einem potentiellen Betreiber und einem tatsächlichen Betreiber stattgefunden hat. Die Deaktivierung der motorisierten Vorrichtung wäre in einem solchen Fall kritisch, denkbar wäre in einem Ausführungsbeispiel, dass ein Alarm ausgelöst wird, z.B. in Form einer Warnlampe oder eines akustischen Warnsignals.

Ein Ausführungsbeispiel weist eine Einrichtung 110 auf, die ausgebildet ist, um eine Identifikation oder Tauglichkeitsinformation über eine Person als potentieller Betreiber eines Kraftfahrzeugs als motorisierte Vorrichtung zu erhalten. Die Einrichtung 120 kann dann verifizieren, ob die Person tatsächlicher Betreiber des Kraftfahrzeugs sein wird, beispielsweise durch Überprüfen, ob sich die Person auf einem Fahrersitz des Kraftfahrzeugs befindet. Ferner kann die Einrichtung 130 dann ausgebildet sein, um das Kfz zu deaktivieren, beispielsweise durch Verhinderung dessen aktiver Teilnahme an einem öffentlichen Verkehr, wenn die Person sich nicht auf dem Fahrersitz befindet oder untauglich ist.

In einem Ausführungsbeispiel entspricht die motorisierte Vorrichtung demnach einem Kraftfahrzeug mit einem Fahrersitz für den tatsächlichen Betreiber, d. h. den Fahrer, und die Einrichtung 120 ist ausgebildet, um zu verifizieren, ob sich der potentielle Betreiber auf dem Fahrersitz befindet. In weiteren Ausführungsbeispielen kommen als motorisierte Vorrichtungen vielerlei Personenbeförderungsmittel und Betriebsmittel in Betracht. Im Bereich der Kraftfahrzeuge wären neben den Autos oder PKW (PKW = Personenkraftwagen) insbesondere Busse, Lkw (Lkw = Lastkraftwagen), Züge, Flugzeuge, Schiffe, Motorräder und nicht zuletzt auch U-Bahnen und Straßenbahnen Realisierungen der motorisierten Vorrichtung. Ferner kommen auch Werkzeuge, wie Kräne, Bagger usw., in Betracht.

In Ausführungsbeispielen kann die Einrichtung 110 ferner ausgebildet sein, um die Information über den potentiellen Betreiber über eine Einrichtung zum Erfassen zu erhalten, wobei die Einrichtung 110 oder die Einrichtung 120 ausgebildet ist, um eine Positionsinformation von der Einrichtung zum Erfassen zu erhalten.

In weiteren Ausführungsbeispielen kann die Vorrichtung 100 ferner eine Einrichtung zum Speichern vorbestimmter potentieller Betreiber aufweisen und die Einrichtung 130 kann ausgebildet sein, um die motorisierte Vorrichtung zu deaktivieren, wenn eine Diskrepanz zwischen dem vorbestimmten potentiellen Betreiber, dem potentiellen Betreiber und dem tatsächlichen Betreiber vorliegt. Insbesondere im Bereich von Kraftfahrzeugen ist es damit möglich, nur eine bestimmte Personengruppe als tatsächliche Betreiber zuzulassen und diese als vorbestimmte potentielle Betreiber bei der Verifikation zu berücksichtigen. Ist eine Identifikation einer Person, z. B. anhand eines optischen Sensors oder anhand eines Fingerabdrucks geschehen, so kann ein Abgleich mit einem Speicher oder einer Datenbank derart erfolgen, dass weder unbefugte noch untaugliche Personen die motorisierte Vorrichtung betreiben.

In weiteren Ausführungsbeispielen kann die Einrichtung 110 ausgebildet sein, um eine Information über einen Mitbetreiber zu erhalten und die Einrichtung zum Speichern kann ferner ausgebildet sein, um Kombinationen aus gültigen tatsächlichen Betreibern und Mitbetreibern zu speichern, wobei auch die Einrichtung 130 ausgebildet sein kann, um die motorisierte Vorrichtung zu deaktivieren, wenn keine gültige Kombination aus tatsächlichem Betreiber und Mitbetreiber vorliegt. Ein solches Ausführungsbeispiel könnte z. B. zum Einsatz kommen, wenn eine Person ein Kraftfahrzeug nur unter Aufsicht führen kann bzw. nur dann dazu berechtigt ist. Nachdem diese Person gemäß der obigen Beschreibung identifiziert wurde, d. h. als tatsächlicher Betreiber identifiziert wurde, kann in diesem Ausführungsbeispiel auch noch eine Identifikation eines Mitbetreibers, z. B. eines Beifahrers, erfolgen. Wenn sich auf dem Beifahrersitz jedoch keine aufsichtsberechtigte Person befindet, so kann die Einrichtung 130 das Kraftfahrzeug deaktivieren.

Die Einrichtung 130 zum Deaktivieren kann ferner ausgebildet sein, um das Kfz zu deaktivieren, wenn die Identifikation der Person nicht in einer Datenbank vorkommt. Die Einrichtung 130 zum Deaktivieren kann beispielsweise ein Starten eines Motors verhindern. Im Bereich der Personenkraftfahrzeuge wäre es denkbar, dass das Starten eines Motors verhindert wird, beispielsweise aber das Starten einer Standheizung usw. nicht. In anderen Ausführungsbeispielen wäre es denkbar, dass das Starten des Motor funktioniert, nicht aber das Einlegen eines Ganges.

In Ausführungsbeispielen wäre es ferner denkbar, gerade im Bereich von luxuriöseren Kfz, dass wenn beispielsweise ein kritischer Alkoholgehalt eines Fahrers festgestellt wird, automatisiert ein Taxiruf angeboten wird. In solchen Ausführungsbeispielen können z. B. Positionsbestimmungsmittel, die in dem Kfz oder auch in einem Mobilfunkgerät vorhanden sein können, zum Einsatz kommen, um die Position des Fahrers bzw. des Fahrzeugs zu bestimmen, und per Knopfdruck ein Taxi an die entsprechende Stelle zu bringen.

Wie bereits oben erwähnt, können Ausführungsbeispiele die Sensorik, die z.B. im Bereich von Kraftfahrzeugen bereits vorhanden ist, ausnutzen, wobei das Hinzufügen weiterer Sensoren oder auch das Ausnutzen bisher nicht berücksichtigter Sensoren denkbar ist. Ferner können Ausführungsbeispiele Steuergeräte wiederverwenden, wobei beispielsweise eine Auswertung der genannten Signale hinzugefügt werden kann.

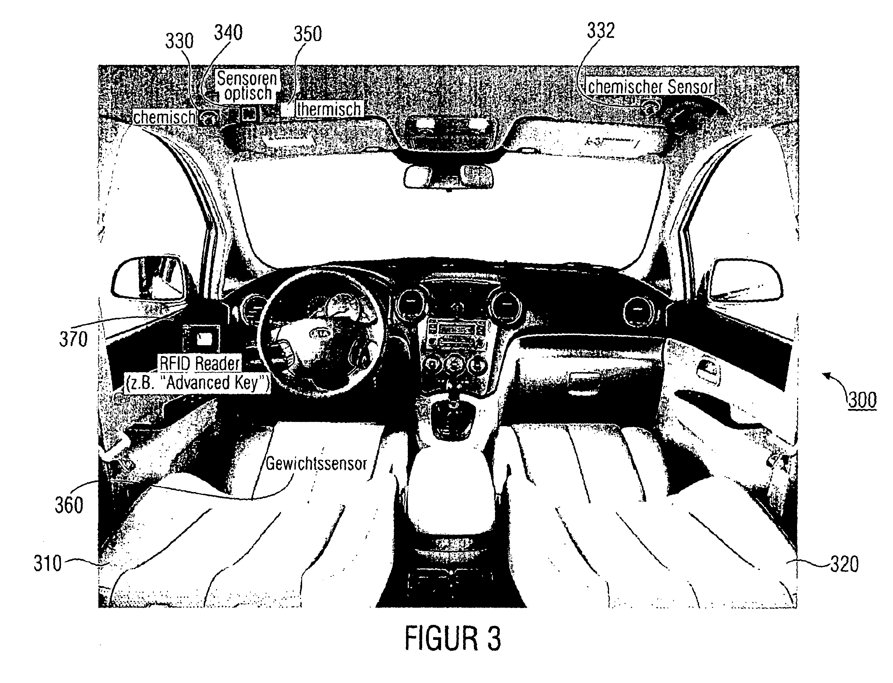

Ein weiteres Ausführungsbeispiel ist in der Fig. 3 dargestellt. Die Fig. 3 zeigt einen Innenraum 300 eines Kraftfahrzeugs mit einem Fahrersitz 310 und einem Beifahrersitz 320. Die Fig. 3 zeigt beispielhaft eine Mehrzahl von Sensoren, die in Ausführungsbeispielen nicht alle vorhanden sein müssen und hier exemplarisch betrachtet werden. Die Fig. 3 zeigt ein Ausführungsbeispiel einer möglichen Anordnung vorkommender Sensoren. Die Fig. 3 zeigt zwei chemische Sensoren 330 und 332, einen optischen Sensor 340, einen thermischen Sensor 350, einen Gewichtssensor 360 und einen RFID-Empfänger 370. In Ausführungsbeispielen können alle diese Sensoren und Komponenten zum Einsatz kommen. Der Aufbau eines Systems kann dabei modular sein und somit eine Vielzahl von Produktvarianten ermöglichen. In dem in Fig. 3 dargestellten Ausführungsbeispiel kann der Gewichtssensor 360 des Fahrersitzes z.B. messen, ob dieser belegt ist. Beispielsweise kann die Messung des Gewichts und der Vergleich mit einem gespeicherten Wert einen ersten Hinweis auf die Identität des Fahrers geben. In diesem Ausführungsbeispiel könnte die weitere Sensorik erst nach Feststellung der Sitzbelegung aktiviert werden.

In dem in Fig. 3 dargestellten Ausführungsbeispiel könnte ferner der RFID-Empfänger 370, der im Folgenden auch RFID-Reader genannt wird, so eingestellt sein, dass er durch Lokalisierung eines Handgeräts detektiert, ob diejenige Person, die sich auf dem Fahrersitz befindet, das Handgerät verwendet oder ob es von einem anderen Insassen oder einer anderen Person benutzt wird. Hier bietet sich in einem Ausführungsbeispiel ein passives, extrem kurz reichweitiges RFID-System an. Erst wenn feststeht, dass der Fahrer das Handgerät benutzt, ist dann eine Freischaltung der Zündung vorgesehen.

In weiteren Ausführungsbeispielen könnten im Bereich eines Kfz-Innenraums auch mehrere herkömmliche RFID-Empfänger vorgesehen sein, wobei durch konventionelle Algorithmik, d. h. durch Kombination der dort empfangenen Signale festgestellt werden kann, von wo aus im Kfz-Innenraum die Signale gesendet wurden. Dabei beschränken sich die Signale nicht zwingend auf elektromagnetische Signale, beispielsweise sind auch Ausführungsbeispiele denkbar, die Infrarot- oder Ultraschallsignale zur Ortsbestimmung eines Handgeräts ausnutzen könnten. Die Freischaltung der Zündung könnte beispielsweise erst dann erfolgen, nachdem feststeht, dass der Atemalkoholgehalt der sich auf dem Fahrersitz 310 befindenden Person einen Grenzwert unterschreitet. Das heißt in diesem Ausführungsbeispiel wäre die Einrichtung 130 zum Deaktivieren derart ausgebildet, dass sie nur unter diesen Voraussetzungen den Betrieb der motorisierten Vorrichtung, hier ein Kraftfahrzeug, zulässt. Die Einrichtung 110 zum Empfangen entspricht in diesem Ausführungsbeispiel einem Steuergerät, das sich hinter dem Armaturenbrett befindet. Die Einrichtung zum Empfangen der Information über den potentiellen Betreiber würde dann z. B. über RFID oder die anderen genannten Schnittstellen bzw. eine Kommunikationsschnittstelle mit dem Handgerät kommunizieren.

Die Einrichtung 120 zum Verifizieren wäre dann z. B. mit dem Gewichtssensor des Fahrersitzes gekoppelt und könnte so bestimmen, dass der Fahrer nach Benutzen des Handgeräts den Fahrersitz 310 nicht mehr verlässt. Dabei könnte der RFID-Reader sicherstellen, dass nur Informationen über einen potentiellen Betreiber der Einrichtung 110 zur Verfügung gestellt werden, die im Bereich des Fahrersitzes 310 gemessen wurden.

In weiteren Ausführungsbeispielen könnte der RFID-Reader auch in bereits bestehende drahtlose Schlüsselsysteme, wie beispielsweise das Audi Advanced Key, kombiniert werden. Hier wäre bereits die Einrichtung 130 zum Deaktivieren in das Fahrzeug integriert.

In weiteren Ausführungsbeispielen könnte ein System auf die Erkennung von mit RIFD ausgerüsteten Führerscheinen und Ausweise erweitert werden. Ausführungsbeispiele dieser Art können dann beispielsweise auch Unfallrisiken, die durch Fahren ohne Fahrerlaubnis hervorgerufen werden, reduzieren, indem Fahrer und Fahrerlaubnis verifiziert werden. Ein Fahrer würde ferne gewarnt bevor er sich ohne gültige Fahrerlaubnis in den Straßenverkehr begibt.

In Ausführungsbeispielen können, wie bereits erwähnt, zusätzliche optische Sensoren über dem Fahrersitz eine optische Identifikation des Fahrers und dessen Verwendung des Handgeräts sicherstellen. In weiteren Ausführungsbeispielen könnten beispielsweise thermische Sensoren oder auch Ultraschallsensoren sicherstellen, dass ein Handgerät sich im Bereich des Fahrersitzes 310 befindet. Thermische Sensoren können in Ausführungsbeispielen dabei ähnlich wie Bewegungsmelder eingesetzt werden.

In weiteren Ausführungsbeispielen wäre die Verwendung von mehreren, beispielsweise chemischen, Sensoren denkbar. In Anlehnung an das Ausführungsbeispiel, welches in der Fig. 3 dargestellt ist, könnte sich ein chemischer Sensor 330 am Holm über dem Fahrersitz 310 befinden. Ein weiterer chemischer Sensor 332 könnte sich am Holm über dem Beifahrersitz 320 befinden. Ein dritter chemischer Sensor wäre beispielsweise hinter dem Fahrersitz 310 denkbar, sowie ein vierter chemischer Sensor hinter dem Beifahrersitz 320.

In diesem Ausführungsbeispiel könnten die chemischen Sensoren zeitaufgelöst Messungen der Alkoholkonzentration in der Atemluft durchführen. Aufgrund der Messungen könnte eine Quelle der Alkoholkonzentration bestimmt werden.

In weiteren Ausführungsbeispielen wäre es denkbar, durch einen biologischen Sensor einen genetischen Fingerabdruck eines Fahrers sowie auch eines Beifahrers zu bestimmen. Anhand der Analyse der Atemluft wäre es auch denkbar zu bestimmen, an welchen genetischen Code, welche Blutalkoholkonzentration geknüpft sein könnte, und so eine entsprechende Detektion durchzuführen. Ausführungsbeispiele in dieser Art würden ferner eine sicherere Identifikation des Fahrers erlauben.

Ausführungsbeispiele der vorliegenden Erfindung tragen somit maßgeblich zu einer Erhöhung der Verkehrssicherheit, sowohl im Straßenverkehr als auch bei anderen Betriebsmitteln, bei.

Insbesondere wird darauf hingewiesen, dass abhängig von den Gegebenheiten das erfindungsgemäße Schema auch in Software implementiert sein kann. Die Implementierung kann auf einem digitalen Speichermedium, insbesondere einer Diskette, einer CD, einer DVD oder einem Flashspeicher mit elektronisch auslesbaren Steuersignalen erfolgen, die so mit einem programmierbaren Computersystem zusammenwirken können, dass das entsprechende Verfahren ausgeführt wird. Allgemein besteht die Erfindung somit auch in einem Computerprogrammprodukt mit auf einem maschinenlesbaren Träger gespeicherten Programmcode zur Durchführung des erfindungsgemäßen Verfahrens, wenn das Computerprogrammprodukt auf einem Rechner abläuft. In anderen Worten ausgedrückt kann die Erfindung somit als ein Computerprogramm mit einem Programmcode zur Durchführung des Verfahrens realisiert werden, wenn das Computerprogrammprodukt auf einem Computer oder Prozessor abläuft.

### Bezugszeichenliste

- 100: Vorrichtung zur Kontrolle
- 110: Einrichtung zum Erhalten
- 120: Einrichtung zum Verifizieren
- 130: Einrichtung zum Deaktivieren

- 300: Kfz-Innenraum
- 310: Fahrersitz
- 320: Beifahrersitz
- 330: Chemischer Sensor
- 332: Chemischer Sensor
- 340: Optischer Sensor
- 350: Thermischer Sensor
- 360: Gewichtssensor
- 370: RFID-Empfänger

## Patentansprüche

1. Vorrichtung (100) zur Kontrolle einer motorisierten Vorrichtung, mit folgenden Merkmalen:
einer Einrichtung (110) zum Erhalten einer Tauglichkeitsinformation über einen potentiellen Betreiber der motorisierten Vorrichtung von einem schnurlosen mobilen Gerät;
einer Einrichtung (120) zum Verifizieren, ob der potentielle Betreiber der motorisierten Vorrichtung ein tatsächlicher Betreiber der motorisierten Vorrichtung sein wird basierend auf einer Positionsbestimmung des mobilen Gerätes und einer Positionsinformation des tatsächlichen Betreibers; und
einer Einrichtung (130) zur Deaktivierung der motorisierten Vorrichtung, wenn eine Diskrepanz zwischen dem potentiellen Betreiber und dem tatsächlichen Betreiber durch die Einrichtung (120) zum Verifizieren festgestellt wird oder wenn die Tauglichkeitsinformation eine Untauglichkeit des potentiellen Betreibers anzeigt.

2. Vorrichtung (100) gemäß Anspruch 1, bei der die Einrichtung (110) ausgebildet ist, um als Tauglichkeitsinformation eine Information über eine Beeinflussung des potentiellen Betreibers durch ein Rauschmittel zu erhalten.

3. Vorrichtung (100) gemäß Anspruch 2, bei der die Einrichtung (110) ausgebildet ist, um als Tauglichkeitsinformation eine Information über eine Beeinflussung durch Alkohol, Drogen oder Medikamente zu erhalten.

4. Vorrichtung (100) gemäß einem der Ansprüche 1 bis 3, bei der die Einrichtung (120) zum Verifizieren ausgebildet ist, um die Positionsinformation über den tatsächlichen Betreiber und eine Positionsinformation über den potentiellen Betreiber in Form der Position des mobilen Gerätes zu erhalten.

5. Vorrichtung (100) gemäß Anspruch 4, bei der die Einrichtung (120) zum Verifizieren ausgebildet ist, um die Positionsinformation des potentiellen Betreibers mit der Positionsinformation des tatsächlichen Betreibers zu vergleichen, und eine Diskrepanz anzuzeigen, wenn die Positionsinformation des potentiellen Betreibers von der Positionsinformation des tatsächlichen Betreibers um mehr als ein vorbestimmtes Maß abweicht.

6. Vorrichtung (100) gemäß einem der Ansprüche 4 oder 5, bei der die Einrichtung (120) zum Verifizieren ausgebildet ist, um die Positionsinformation über den tatsächlichen Betreiber über einen ersten Sensor zu erhalten.

7. Vorrichtung gemäß einem der Ansprüche 1 bis 6, bei der die Einrichtung (110) ausgebildet ist, um die Tauglichkeitsinformation von einem zweiten Sensor zu erhalten.

8. Vorrichtung gemäß einem der Ansprüche 6 oder 7, bei der der erste oder der zweite Sensor einen thermischen Sensor, einen Gewichtssensor, einen Drucksensor, einen optischen Sensor, einen chemischen Sensor, einen Fingerabdrucksensor, einen genetischen Sensor, oder einen biologischen Sensor umfasst.

9. Vorrichtung (100) gemäß einem der Ansprüche 1 bis 8, bei der die Einrichtung (110) ausgebildet ist, um die Information über RFID (RFID = Radio Frequency Identification, Hochfrequenzidentifikation), Bluetooth, IR (IR = Infrared, Infrarot) oder Ultraschall zu erhalten.

10. Vorrichtung (100) gemäß einem der Ansprüche 1 bis 9, bei der die motorisierte Vorrichtung als Kraftfahrzeug mit einem Fahrersitz für den tatsächlichen Betreiber ausgebildet ist und die Einrichtung (120) ausgebildet ist, um zu verifizieren, ob sich der potentielle Betreiber auf dem Fahrersitz befindet.

11. Vorrichtung (100) gemäß einem der Ansprüche 1 bis 10, die ferner eine Einrichtung zum Speichern von vorbestimmten potentiellen Betreibern aufweist und die Einrichtung (130) ausgebildet ist, um die motorisierte Vorrichtung zu deaktivieren, wenn eine Diskrepanz zwischen dem vorbestimmten potentiellen Betreiber, dem potentiellen Betreiber und dem tatsächlichen Betreiber vorliegt.

12. Vorrichtung (100) gemäß Anspruch 11, bei der die Einrichtung (110) ausgebildet ist, um eine Information über einen Mitbetreiber zu erhalten und die Einrichtung zum Speichern ferner ausgebildet ist, um Kombinationen aus gültigem tatsächlichen Betreiber und Mitbetreiber zu speichern und die Einrichtung (130) ausgebildet ist, um die motorisierte Vorrichtung zu deaktivieren, wenn keine gültige Kombination aus tatsächlichem Betreiber und Mitbetreiber vorliegt.

13. Vorrichtung (100) gemäß einem der Ansprüche 1 bis 12, bei der die Einrichtung (110) ausgebildet ist, um eine Tauglichkeitsinformation einer Person, als potentiellem Betreiber, eines Kraftfahrzeugs, als motorisierte Vorrichtung, von einem Alkomaten, als mobilem schnurlosen Gerät zu erhalten, bei der die Einrichtung (120) ausgebildet ist, um zu verifizieren, ob die Person tatsächlicher Betreiber des Kraftfahrzeugs sein wird, durch Überprüfen, ob sich die Person auf einem Fahrersitz des Kraftfahrzeugs befindet und der Alkomat von dieser Person bedient wird, und wobei die Einrichtung (130) ausgebildet ist, um das Kraftfahrzeug zu deaktivieren, durch Verhinderung dessen aktiver Teilnahme an einem Verkehr, wenn die Person sich nicht auf dem Fahrersitz befindet oder untauglich ist.

14. Vorrichtung (100) gemäß Anspruch 13, wobei die Einrichtung (130) ausgebildet ist, um das Kraftfahrzeug zu deaktivieren, wenn eine Identifikation der Person nicht in einer Datenbank vorkommt.

15. Mobiles Gerät zur Erfassung einer Tauglichkeitsinformation eines potentiellen Betreibers mit einer Schnittstelle zur schnurlosen Kommunikation der Tauglichkeitsinformation, bei dem die Schnittstelle zur Kommunikation über RFID, Bluetooth, Infrarot oder Ultraschall ausgebildet ist, wobei das mobile Gerät ferner einen Probekörper aufweist, anhand dessen sich das mobile Gerät in einem erfassten Sensorsignal verifizieren bzw. identifizieren lässt.

16. Mobiles Gerät gemäß Anspruch 15, das einen Fingerabdrucksensor aufweist.

17. Verfahren zur Kontrolle einer motorisierten Vorrichtung, mit folgenden Schritten:
Erfassen einer Tauglichkeitsinformation eines potentiellen Betreibers mit einem mobilen Gerät;
Erhalten der Tauglichkeitsinformation über den potentiellen Betreiber schnurlos von dem mobilen Gerät;
Verifizieren, ob der potentielle Betreiber der motorisierten Vorrichtung ein tatsächlicher Betreiber der motorisierten Vorrichtung sein wird basierend auf einem schnurlosen Bestimmen der Position des mobilen Gerätes und einer Positionsinformation des tatsächlichen Betreibers; und
Deaktivieren der motorisierten Vorrichtung, wenn eine Diskrepanz zwischen dem potentiellen Betreiber und dem tatsächlichen Betreiber festgestellt wird oder wenn die Tauglichkeitsinformation eine Untauglichkeit des potentiellen Betreibers anzeigt.

18. Computerprogramm mit einem Programmcode zur Durchführung des Verfahrens gemäß Anspruch 17, wenn der Programmcode auf einem Prozessor ausgeführt wird.

## Claims

1. An apparatus (100) for checking a motorized device, comprising:
a means (110) for obtaining aptitude information about a potential operator of the motorized device from a wireless mobile device;
a means (120) for verifying whether the potential operator of the motorized device will be an actual operator of the motorized device on the basis of determining the position of the mobile device and of positional information about the actual operator; and
a means (130) for deactivating the motorized device when a discrepancy between the potential operator and the actual operator is established by the means (120) for verifying or when the aptitude information indicates an inaptitude of the potential operator.

2. Apparatus (100) as claimed in claim 1, wherein the means (110) is configured to obtain, as aptitude information, information about the potential operator being influenced by an intoxicant.

3. Apparatus (100) as claimed in claim 2, wherein the means (110) is configured to obtain, as aptitude information, information about any influence by alcohol, drugs or medication.

4. Apparatus (100) as claimed in any of claims 1 to 3, wherein the means (120) for verifying is configured to obtain the positional information about the actual operator and positional information about the potential operator in the form of the position of the mobile device.

5. Apparatus (100) as claimed in claim 4, wherein the means (120) for verifying is configured to compare the positional information about the potential operator with the positional information about the actual operator, and to indicate a discrepancy if the positional information about the potential operator deviates from the positional information about the actual operator by more than a predetermined measure.

6. Apparatus (100) as claimed in one of claims 4 or 5, wherein the means (120) for verifying is configured to obtain the positional information about the actual operator via a first sensor.

7. Apparatus as claimed in any of claims 1 to 6, wherein the means (110) is configured to obtain the aptitude information from a second sensor.

8. Apparatus as claimed in one of claims 6 or 7, wherein the first or the second sensor comprises a thermal sensor, a weight sensor, a pressure sensor, an optical sensor, a chemical sensor, a fingerprint sensor, a genetic sensor, or a biological sensor.

9. Apparatus (100) as claimed in any of claims 1 to 8, wherein the means (110) is configured to obtain the information via RFID (radio frequency identification), Bluetooth, IR (infrared), or ultrasound.

10. Apparatus (100) as claimed in any of claims 1 to 9, wherein the motorized device is configured as a motor vehicle comprising a driver's seat for the actual operator, and the means (120) is configured to verify whether the potential operator is seated on the driver's seat.

11. Apparatus (100) as claimed in any of claims 1 to 10, further comprising a means for storing predetermined potential operators, and wherein the means (130) is configured to deactivate the motorized device when there is a discrepancy between the predetermined potential operator, the potential operator, and the actual operator.

12. Apparatus (100) as claimed in claim 11, wherein the means (110) is configured to obtain information about a co-operator, and the means for storing is further configured to store combinations of the validated actual operator and the co-operator, and the means (130) is configured to deactivate the motorized device when there is no valid combination of the actual operator and the co-operator.

13. Apparatus (100) as claimed in any of claims 1 to 12, wherein the means (110) is configured to obtain aptitude information about a person as a potential operator, about a motor vehicle as a motorized device, about a breathalyzer as a mobile wireless device, wherein the means (120) is configured to verify whether the person will be the actual operator of the motor vehicle by verifying whether the person is seated on a driver's seat of the motor vehicle and the breathalyzer is operated by said person, and wherein the means (130) is configured to deactivate the motor vehicle by preventing its active participation in traffic if the person is not seated on the driver's seat or is inapt.

14. Apparatus (100) as claimed in claim 13, wherein the means (130) is configured to deactivate the motor vehicle if an identification of the person does not appear in a database.

15. Mobile device for detecting aptitude information about a potential operator, comprising an interface for wireless communication of the aptitude information, wherein the interface is configured for communication via RFID, Bluetooth, infrared or ultrasound, the mobile device further comprising a test piece by means of which the mobile device may be verified and/or identified in a detected sensor signal.

16. Mobile device as claimed in claim 15, comprising a fingerprint sensor.

17. Method of checking a motorized device, comprising:
detecting aptitude information about a potential operator using a mobile device;
obtaining the aptitude information about the potential operator from the mobile device in a wireless manner;
verifying whether the potential operator of the motorized device will be an actual operator of the motorized device on the basis of wirelessly determining the position of the mobile device and positional information about the actual operator; and
deactivating the motorized device when a discrepancy is established between the potential operator and the actual operator or when the aptitude information indicates an inaptitude of the potential operator.

18. Computer program comprising a program code for performing the method as claimed in claim 17, when the program code is executed on a processor.

## Revendications

1. Dispositif (100) de contrôle d'un dispositif motorisé, aux caractéristiques suivantes:
un moyen (110) pour obtenir d'un dispositif mobile sans fil une information d'aptitude sur un utilisateur du dispositif motorisé;
un moyen (120) pour vérifier si l'utilisateur potentiel du dispositif motorisé sera un utilisateur réel du dispositif motorisé sur base d'une détermination de position du dispositif mobile et d'une information de position de l'utilisateur réel; et
un moyen (130) pour désactiver le dispositif motorisé s'il est constaté par le moyen (120) pour vérifier une différence entre l'utilisateur potentiel et l'utilisateur réel ou si l'information d'aptitude indique une inaptitude de l'utilisateur potentiel.

2. Dispositif (100) selon la revendication 1, dans lequel le moyen (110) est réalisé pour obtenir, comme information d'aptitude, une information sur une influence sur l'utilisateur potentiel par un moyen de bruit.

3. Dispositif (100) selon la revendication 2, dans lequel le moyen (110) est réalisé pour obtenir, comme information d'aptitude, une information sur une influence par l'alcool, des drogues ou des médicaments.

4. Dispositif (100) selon l'une des revendications 1 à 3, dans lequel le moyen (120) pour vérifier est réalisé pour obtenir l'information de position sur l'utilisateur réel et une information de position sur l'utilisateur potentiel sous forme de position du dispositif mobile.

5. Dispositif (100) selon la revendication 4, dans lequel le moyen (120) pour vérifier est réalisé pour comparer l'information de position de l'utilisateur potentiel avec l'information de position de l'utilisateur réel, et pour indiquer une différence lorsque l'information de position de l'utilisateur potentiel diffère de plus d'une mesure prédéterminée de l'information de position de l'utilisateur réel.

6. Dispositif (100) selon l'une des revendications 4 ou 5, dans lequel le moyen (120) pour vérifier est réalisé pour obtenir l'information de position sur l'utilisateur réel par l'intermédiaire d'un premier capteur.

7. Dispositif selon l'une des revendications 1 à 6, dans lequel le moyen (110) est réalisé pour obtenir l'information d'aptitude d'un deuxième capteur.

8. Dispositif selon l'une des revendications 6 ou 7, dans lequel le premier ou le deuxième capteur comporte un capteur thermique, un capteur de poids, un capteur de pression, un capteur optique, un capteur chimique, un capteur d'empreinte digitale, un capteur génétique ou un capteur biologique.

9. Dispositif (100) selon l'une des revendications 1 à 8, dans lequel le moyen (110) est réalisé pour obtenir l'information via RFID (RFID = Radio Frequency Identification, identification à haute fréquence), Bluetooth, IR (IR = Infrared, infrarouge) ou ultrasons.

10. Dispositif (100) selon l'une des revendications 1 à 9, dans lequel le dispositif motorisé est réalisé sous forme de véhicule à moteur avec un siège de conducteur pour l'utilisateur réel et le moyen (120) est réalisé pour vérifier si l'utilisateur potentiel se trouve sur le siège de conducteur.

11. Dispositif (100) selon l'une des revendications 1 à 10, présentant par ailleurs un moyen pour mémoriser des utilisateurs potentiels prédéterminés et le moyen (130) étant réalisé pour désactiver le dispositif motorisé lorsqu'une différence entre l'utilisateur potentiel prédéterminé, l'utilisateur potentiel et l'utilisateur réel est présente.

12. Dispositif (100) selon la revendication 11, dans lequel le moyen (110) est réalisé pour obtenir une information sur un co-utilisateur et le moyen pour mémoriser est par ailleurs réalisé pour mémoriser des combinaisons d'utilisateur réel valable et de co-utilisateur et le moyen (130) est réalisé pour désactiver le dispositif motorisé lorsqu'il n'est pas présent de combinaison valable d'utilisateur réel et de co-utilisateur.

13. Dispositif (100) selon l'une des revendications 1 à 12, dans lequel le moyen (110) est réalisé pour obtenir une information d'aptitude d'une personne, comme utilisateur potentiel d'un véhicule à moteur, comme dispositif motorisé, d'un alcomat, comme dispositif mobile sans fil, dans lequel le moyen (120) est réalisé pour vérifier si la personne sera l'utilisateur réel du véhicule à moteur, en vérifiant si la personne se trouve sur un siège de conducteur du véhicule à moteur et que l'alcomat est utilisé par cette personne, et le moyen (130) est réalisé pour désactiver le véhicule à moteur en empêchant sa participation active à un trafic lorsque la personne ne se trouve pas sur le siège de conducteur ou n'est pas apte.

14. Dispositif (100) selon la revendication 13, dans lequel le moyen (130) est réalisé pour désactiver le véhicule à moteur lorsqu'une identification de la personne n'est pas présente dans une banque de données.

15. Dispositif mobile pour saisir une information d'aptitude d'un utilisateur potentiel avec une interface de communication sans fil de l'information d'aptitude, dans lequel l'interface est réalisée pour la communication via RFID, Bluetooth, infrarouge ou ultrasons, le dispositif mobile présentant par ailleurs un corps de test à l'aide duquel le dispositif mobile peut être vérifié ou identifié par un signal de capteur détecté.

16. Dispositif mobile selon la revendication 15, présentant un capteur d'empreinte digitale.

17. Procédé de contrôle d'un dispositif motorisé, aux étapes suivantes consistant à:
capter une information d'aptitude d'un utilisateur potentiel à l'aide d'un dispositif mobile;
obtenir l'information d'aptitude sur l'utilisateur potentiel sans fil du dispositif mobile;
vérifier si l'utilisateur potentiel du dispositif motorisé sera un utilisateur réel du dispositif motorisé sur base d'une détermination sans fil de la position du dispositif mobile et d'une information de position de l'utilisateur réel; et
désactiver le dispositif motorisé lorsqu'il est constaté une différence entre l'utilisateur potentiel et l'utilisateur réel ou lorsque l'information d'aptitude indique une inaptitude de l'utilisateur potentiel.

18. Programme d'ordinateur avec un code de programme pour réaliser le procédé selon la revendication 17 lorsque le code de programme est exécuté sur un processeur.
